# EUROPÄISCHE PATENTANMELDUNG

(11) **EP 0 681 848 A1**
(43) Veröffentlichungstag der Anmeldung: **15.11.1995**
(21) Anmeldenummer: 95107200.8
(22) Anmeldetag: 12.05.1995
(51) Int. Cl.: A61M 27/00

(54) **Instrumentarium zum Einziehen eines Drainageschlauches in eine Schaumstoffplatte**

(30) Priorität: 13.05.1994 DE 9407964 U
(71) Anmelder: Arnold, Axel, D-78606 Seitingen (DE)
(72) Erfinder: Arnold, Axel, D-78606 Seitingen (DE)
(74) Vertreter: Patentanwälte Westphal, Buchner, Mussgnug Neunert, Göhring

(57) **Zusammenfassung**

Es wird ein Intrumentarium beschrieben, mit welchem ein Drainageschlauch flächenparallel in eine Schaumstoffplatte für die Wunddrainage eingezogen werden kann. Die Schaumstoffplatte wird mittels einer Halterung (18, 20) vor einem in der Einziehrichtung verlaufenden Führungsrohr (10) gehalten. Der Drainageschlauch wird auf das Ende eines Spießes (34) geschraubt und mittels des Spießes (34) durch das Führungsrohr (10) eingezogen. Der Spieß (34) ist lösbar an einem Griff (44) befestigt.

## Beschreibung

Die Erfindung betrifft ein Intrumentarium zum flächenparallelen Einziehen eines Drainageschlauches in eine Schaumstoffplatte gemäß dem Oberbegriff des Anspruchs 1.

Bei der Versorgung tiefer Wunden wird eine kontinuierliche Saugdrainage (Redon-Drainage) verwendet, bei welcher ein Drainageschlauch mit seinem perforierten Ende in die Wunde eingelegt wird, um das Wundsekret und dergleichen durch Unterdruck abzusaugen. Für die Behandlung großflächiger offener Wunden ist es bekannt (WO 93/09727), eine Schaumstoffplatte, z. B. aus einem Polyvinylalkohol-Schaumstoff, entsprechend der Wundfläche zuzuschneiden und in die Wundtasche einzulegen. Der Drainageschlauch wird flächenparallel in diese Schaumstoffplatte eingezogen, so daß sein perforiertes Ende in der Schaumstoffplatte liegt. Dann wird das Wundgebiet mit der Schaumstoffplatte mit einer Folie abgedeckt und vakuumversiegelt. Über den transcutan ausgeleiteten Drainageschlauch wird das Saugvakuum angelegt, wobei über die poröse Schaumstoffplatte eine gleichmäßig verteilte großflächige Saugwirkung erreicht wird.

Um den Drainageschlauch in die Schaumstoffplatte einzuziehen, wird ein Spieß verwendet der an seinem hinteren Ende einen Gewindeansatz aufweist, auf welchen der Drainageschlauch aufgeschraubt wird. Mit diesem Spieß wird die Schaumstoffplatte flächenparallel durchstoßen, um den Drainageschlauch einzuziehen. Wegen der weichen nachgiebigen Struktur der Schaumstoffplatte und wegen der geringen Stärke der Schaumstoffplatte ist das flächenparalle Durchstoßen der Schaumstoffplatte mittels des Spießes schwierig. In der Regel sind hierzu zwei Personen erforderlich, von denen eine den Spieß hält und führt, während die andere die Schaumstoffplatte hält und ein Ausweichen der Schaumstoffplatte vor der Spitze des Spießes verhindert. Das Einziehen des Drainageschlauches ist daher mühsam, personalintensiv und erfordert Übung. Außerdem besteht eine nicht unerhebliche Verletzungsgefahr beim Durchstoßen der Schaumstoffplatte mittels der scharfen Spitze des Spießes.

Der Erfindung liegt die Aufgabe zugrunde, ein Instrumentarium zum flächenparallelen Einziehen des Drainageschlauches in die Schaumstoffplatte zur Verfügung zu stellen, welches eine einfache und zuverlässige Handhabung ermöglicht und die Verletzungsgefahr minimiert.

Diese Aufgabe wird erfindungsgemäß gelöst durch ein Instrumentarium mit den Merkmalen des Anspruchs 1.

Vorteilhafte Ausführungsformen der Erfindung sind in den Unteransprüchen angegeben.

Bei dem erfindungsgemäßen Instrumentarium wird die Schaumstoffplatte mittels einer Halterung festgelegt und vor einem Führungsrohr positioniert. Der Spieß mit dem Drainageschlauch wird durch dieses Führungsrohr geschoben, so daß er zwangsgeführt in die Schaumstoffplatte eintritt und die in der Halterung festgelegte Schaumstoffplatte exakt flächenparallel in der gewünschten Einziehrichtung durchbohrt. Da die Schaumstoffplatte in der Halterung festgelegt ist, ist ein seitliches Ausweichen der weichen nachgiebigen Schaumstoffplatte vor der Spitze des Spießes nicht möglich. Es ist somit keine zweite Person erforderlich, die die Schaumstoffplatte hält und die Bahn der Spitze des Spießes durch die Schaumstoffplatte kontrolliert. Der Drainageschlauch kann von einer einzigen Person eingezogen werden, die mit einer Hand das Führungsrohr mit der Halterung hält und mit der anderen Hand den Spieß mit dem Drainageschlauch durch das Führungsrohr hindurchschiebt, um die Schaumstoffplatte zu durchbohren. Die Halterung der Schaumstoffplatte und die Zwangsführung des Spießes bezüglich der festgehaltenen Schaumstoffplatte gewährleisten ein exaktes Einziehen des Drainageschlauches, ohne daß es hierzu einer besonderen Geschicklichkeit oder Übung bedarf. Da die Schaumstoffplatte nicht manuell vor der Spitze des Spießes gehalten werden muß, ist auch die Gefahr einer Verletzung beim Einziehen des Draingeschlauches erheblich verringert.

In einer zweckmäßigen Ausführung erfaßt die Halterung die Schaumstoffplatte von der Oberseite und der Unterseite über die gesamte Länge des Einziehweges, so daß die weiche Schaumstoffplatte über die gesamte Länge des Weges, in welchem die Spitze des Spießes den Schaumstoff durchdringt, festgehalten ist.

Vorzugsweise besteht die Halterung aus zwei Rohrschalen, die sich von der Oberseite und der Unterseite an die Schaumstoffplatte anlegen. Die beiden aneinander anliegenden Axialkanten der Rohrschalen klemmen dabei die Schaumstoffplatte beiderseits der Einziehbahn, während im Inneren der beiden Rohrschalen der Schaumstoff nicht komprimiert wird, so daß das Durchstoßen des Schaumstoffes mittels des Spießes nicht erschwert ist.

Das Schließen und Klemmen der Halterung erfolgt zweckmäßig durch einen Scherengriff, der gleichzeitig zum Festhalten des Führungsrohres und der Halterung dient.

Um die Handhabung des Spießes zu erleichtern und sicherer zu machen, ist vorzugsweise ein Griff mit einer axial durchgehenden Bohrung vorgesehen, durch welche der Spieß mit dem Drainageschlauch hindurchgeführt werden kann. Der Spieß weist an seinem hinteren Ende einen Einstich auf, in welchen eine Verriegelung des Griffes eingreift, so daß der Griff am hinteren Ende des Spießes axial festgelegt werden kann. Der Griff ermöglicht ein bequemes und ergonomisch günstiges Erfassen und Handhaben des Spießes beim Durchstoßen der Schaumstoffplatte. Sobald die Schaumstoffplatte durchstoßen ist, wird die Verriegelung des Griffes gelöst, so daß der Griff nach hinten über den Drainageschlauch von dem Spieß abgezogen werden kann.

Vorzugsweise ist auch hinter der Spitze des Spießes ein Einstich vorgesehen. Der vom hinteren Ende des Spießes abgezogene Griff kann von vorn auf die aus der durchstoßenen Schaumstoffplatte herausragende Spitze aufgesteckt und an diesem Einstich verriegelt werden. Der Spieß kann dann bequem und sicher mittels des Griffes vollständig durch die Schaumstoffplatte hindurch und aus dieser herausgezogen werden, bis der Drainageschlauch vollständig in der Schaumstoffplatte positioniert ist und der Spieß von dem Drainageschlauch abgeschraubt werden kann. Da sich die scharfe Spitze des Spießes dabei im Inneren des Griffes befindet, ist jegliche Verletzungsgefahr durch die scharfe Spitze des Spießes ausgeschlossen.

Im folgenden wird die Erfindung anhand eines in der Zeichnung dargestellten Ausführungsbeispiels näher erläutert. Es zeigen:
- Figur 1: eine Seitenansicht des Führungsrohres mit der Halterung, wobei die eine Rohrschale der Halterung teilweise weggebrochen ist,
- Figur 2: eine Stirnansicht gemäß dem Pfeil X in Figur 1,
- Figur 3: eine Ansicht des Führungsrohres mit der Halterung von oben,
- Figur 4: den Spieß,
- Figur 5: eine teilweise axial geschnittene Seitenansicht des Griffes und
- Figur 6: einen Schnitt längs der Linie A-A in Figur 5.

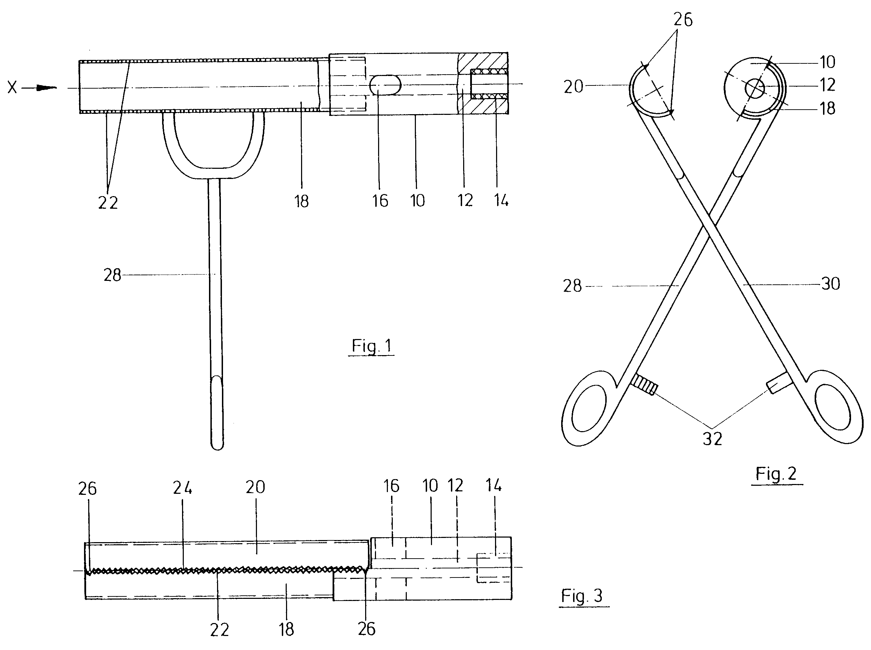

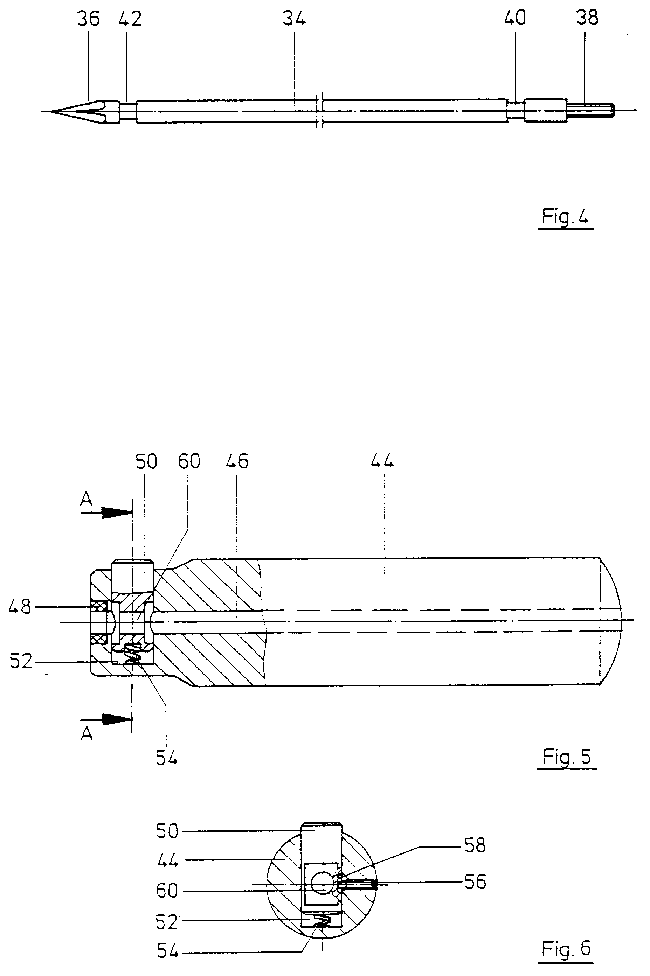

Das Instrumentarium zum Einziehen eines Drainageschlauches in eine Schaumstoffplatte besteht aus dem Führungsrohr mit Halterung, das in den Figuren 1 bis 3 dargestellt ist, dem Spieß, der in Figur 4 dargestellt ist, und dem Griff, der in den Figuren 5 und 6 dargestellt ist.

Wie die Figuren 1 bis 3 zeigen, ist ein Führungsrohr 10, vorzugsweise aus Edelstahl, mit einer axial durchgehenden Innenbohrung 12 vorgesehen. Der Durchmesser der Innenbohrung 12 ist so gewählt, daß der später beschriebene Spieß mit dem Draingeschlauch geführt durch die Innenbohrung 12 geschoben werden kann. In das rückwärtige Ende des Führungsrohres ist eine Kunststoffbuchse 14 eingesetzt, die die Wandung der Innenbohrung 12 auskleidet, um eine Beschädigung der scharfen Spitze des Spießes beim Einführen in die Innenbohrung 12 zu verhindern. Hinter seinem vorderen Ende wird das Führungsrohr 10 diametral von einem durchgehenden Fenster 16 durchsetzt.

An das vordere Ende des Führungsrohres 10 schließt sich eine Halterung für eine nicht dargestellte Schaumstoffplatte an. Die Halterung besteht aus zwei längs einer axialen Mittelebene getrennten halbzylindrischen Rohrschalen 18 und 20. Der Außendurchmesser der dünnwandigen Rohrschalen 18 und 20 entspricht dem Außendurchmesser des Führungsrohres 10. Der Innendurchmesser der Rohrschalen 18 und 20 entspricht im wesentlichen der Stärke der Schaumstoffplatte. Die erste Rohrschale 18 ist als einstückige Verlängerung des Führungsrohres 10 ausgebildet. Die zweite Rohrschale 20 ist vollständig von dem Führungsrohr 10 und der ersten Rohrschale 18 getrennt und gegen diese bewegbar. Die beiden Rohrschalen 18 und 20 weisen die gleiche axiale Länge auf, so daß die zweite Rohrschale 20 mit ihren Längskanten 24 an die Längskanten 22 der ersten Rohrschale 18 angelegt werden kann, wodurch die beiden Rohrschalen 18 und 20 eine rohrförmige axial fluchtende Verlängerung des Führungsrohres 10 bilden.

Die beiden aneinander zur Anlage kommenden Längskanten 22 und 24 der beiden Rohrschalen 18 und 20 sind mit einer Verzahnung versehen, deren Zahnspitzen gegen das Führungsrohr 10 hin geneigt sind. Darüber hinaus greifen die Längskanten 22 und 24 mit vorspringenden Zähnen 26 und entsprechenden Aussparungen ineinander. Solche Zähne 26 an den Längskanten 24 und entsprechende Ausnehmungen an den Längskanten 22 sind zumindest am vorderen und hinteren Ende der Längskanten 22 bzw. 24 vorgesehen. Gegebenenfalls sind weitere Zähne über die Längserstreckung der Längskanten 22 und 24 verteilt angeordnet.

Zur Betätigung der Halterung dient ein Scherengriff, an dessen einer Branche 28 über eine Gabel die erste Rohrschale 18 und das Führungsrohr 10 befestigt sind und an deren anderer Branche 30 über eine entsprechende Gabel die zweite Rohrschale 20 befestigt ist. Die Schwenkebene des Scherengriffes liegt senkrecht zur Achse des Führungsrohres 10. Die Betätigung des Scherengriffes erfolgt in üblicher Weise über Fingerringe. Beim Schließen des Scherengriffes wird die zweite Rohrschale 20 auf die erste Rohrschale 18 geschwenkt, so daß die Längskante 22 und 24 aneinander zur Anlage kommen. In der Schließstellung kann der Scherengriff über eine an den Branchen 28 und 30 angebrachte Rastsperre 32 lösbar arretiert werden.

Der in Figur 4 gezeigte Spieß 34 besteht aus einem geraden Stahlschaft mit rundem Querschnitt, an dem vorne eine scharfe Spitze 36 angeschliffen ist. Am hinteren Ende ist der Schaft abgedreht und mit einem Außengewinde versehen. Der so gebildete Gewindeansatz 38 kann in einen Kunststoff-Drainageschlauch eingeschraubt werden. Der Gewindedurchmesser des Gewindeansatzes 38 ist so auf den Innendurchmesser des Drainageschlauches abgestimmt, daß der aufgeschraubte Drainageschlauch zuverlässig am Ende des Spießes 34 gehalten wird. Der Außendruchmesser des Drainageschlauches entspricht dem Außendurchmesser des Spießes 34.

Vor dem mit dem Gewindeansatz 38 versehenen hinteren Ende des Spießes 34 ist ein Einstich 40 eingearbeitet. Ein entsprechender Einstich 42 ist hinter der Spitze 36 in den Schaft des Spießes 34 eingearbeitet.

Der in den Figuren 5 und 6 dargestellte Griff 44 hat eine langgestreckte im wesentlichen zylindrische Form und besteht aus Kunststoff, Holz, Metall oder einem sonstigen geeigneten Werkstoff. Der Griff 44 wird koaxial von einer durchgehenden Bohrung 46 durchsetzt. Der Durchmesser der Bohrung 46 ist (ebenso wie der Durchmesser der Innenbohrung 12 des Führungsrohres 10) so gewählt, daß der Spieß 34 mit dem Drainageschlauch geführt durch die Bohrung 46 geschoben werden kann. Das vordere Ende der Bohrung 46 ist durch eine Kunststoffbuchse 48 ausgekleidet.

Hinter dem vorderen Ende des Griffes 44 ist eine Verriegelung vorgesehen. Die Verriegelung besteht aus einem Bolzen 50, der diametral verschiebbar in einer Sackbohrung 52 des Griffes 44 gelagert ist. Eine Druckfeder 54 ist zwischen den Boden der Sackbohrung 52 und die innere Stirnfläche des Bolzens 50 eingesetzt und drückt den Bolzen 50 radial nach außen. Der Verschiebungsweg des Bolzens 50 in der Sackbohrung 52 ist begrenzt durch eine Madenschraube 56, die senkrecht zu dem Bolzen 50 radial in den Griff 44 eingeschraubt ist und in eine den Verschiebungsweg bestimmende Längsnut 58 des Bolzens 50 eingreift. Der Bolzen 50 weist eine Querbohrung 60 auf, deren Durchmesser im wesentlichen mit dem Durchmesser der Bohrung 46 übereinstimmt. Die Achse der Querbohrung 60 verläuft parallel zur Achse der Bohrung 46, wobei die in die Längsnut 58 eingreifende Madenschraube 56 als Verdrehsicherung des Bolzens 50 wirkt und die Parallelität der Querbohrung 60 mit der Bohrung 46 gewährleistet. Wird der Bolzen 50 durch radialen Druck auf sein äußeres aus dem Griff 44 herausragendes Ende gegen die Kraft der Druckfeder 54 nach innen gedrückt, so wird die Querbohrung 60 des Bolzens 50 mit der Bohrung 46 des Griffes 44 in axiale Fluchtung gebracht. Wird der Bolzen 50 freigegeben, so drückt die Druckfeder 54 den Bolzen 50 radial nach außen, in seine durch die Längsnut 58 vorgegebene Endstellung, in welcher die Querbohrung 60 gegen die Bohrung 46 versetzt ist und nicht mehr mit dieser fluchtet. Im Bereich der Querbohrung 60 ist der Bolzen 50 auf eine Breite abgeflacht, die der axialen Breite der Einstiche 40 und 42 entspricht.

Zum Einziehen eines Drainageschlauches in eine Schaumstoffplatte wird das Instrumentarium in folgender Weise verwendet:
Der nicht dargestellte Drainageschlauch wird mit seinem perforierten Ende auf den Gewindeansatz 38 des Spießes 34 geschraubt.

Die nicht dargestellte Schaumstoffplatte wird mit dem Bereich, in welchen der Drainageschlauch eingezogen werden soll, zwischen die auseinandergespreizten Rohrschalen 18 und 20 gelegt. Durch Schließen des Scherengriffes werden die Rohrschalen 18 und 20 gegeneinander bewegt, so daß die Schaumstoffplatte zwischen den Längskanten 22 und 24 festgeklemmt wird. In der Klemmstellung werden die Rohrschalen 18 und 20 mittels der Rastsperre 32 arretiert. Der weiche nachgiebige Schaumstoff der Schaumstoffplatte wird zwischen den Längskanten 22 und 24 zusammengequetscht, wobei die Verzahnung der Längskanten 22 und 24 und die Zähne 26 die Schaumstoffplatte festhalten. Der im Inneren zwischen den Rohrschalen 18 und 20 liegende Bereich der Schaumstoffplatte wird dabei nicht zusammengequetscht.

Der Spieß 34 mit dem Drainageschlauch wird von hinten in die Bohrung 46 des Griffes 44 eingeführt. Dabei wird der Bolzen 50 niedergedrückt, so daß seine Querbohrung 60 mit der Bohrung 46 des Griffes 44 fluchtet. Der Spieß kann auf diese Weise unbehindert durch den Griff 44 geführt werden, bis die Spitze 36 vorn aus dem Griff 44 herausragt und sich der Bolzen 50 im mittleren Schaftbereich des Spießes 34 befindet. Nun wird der Bolzen 50 freigegeben. Der Spieß 34 wird nach vorn aus dem Griff 44 herausgezogen. Wenn der hintere Einstich 40 an den Bolzen 50 gelangt, rastet der Bolzen 50 unter dem Druck der Druckfeder 54 in den hinteren Einstich 40 ein und der Spieß 34 ist axial in dem Griff 44 verriegelt.

Nun wird der Spieß 34 von hinten in die Innenbohrung 12 des Führungsrohres 10 eingeführt. Wenn die Spitze 36 des Spießes 34 vorn aus dem Führungsrohr 10 austritt, dringt sie in die Schmalseite der zwischen den Rohrschalen 18 und 20 eingeklemmten Schaumstoffplatte ein. Der Spieß 34 kann mittels des Griffes 44 vorgeschoben werden, so daß sich die Spitze 36 durch die Schaumstoffplatte bohrt. Die die Schaumstoffplatte klemmenden Längskanten 22 und 24 mit ihrer Zahnung und den scharfen vorspringenden Zähnen 26 halten dabei die Schaumstoffplatte fest und erzeugen den Gegendruck für die die Schaumstoffplatte durchbohrende Spitze 36.

Wenn die Spitze 36 die gesamte Länge der Schaumstoffplatte durchbohrt hat und am Ende der durch die Rohrschalen 18 und 20 gebildeten Halterung austritt, wird der Bolzen 50 wieder gegen die Druckfeder 54 niedergedrückt, so daß der Bolzen 50 aus dem Einstich 40 des Spießes 34 herausgehoben wird. Nun wird der Griff 44 über das hintere Ende des Spießes 34 und den aufgeschraubten Drainageschlauch nach hinten abgezogen. Der Griff 44 wird dann in umgekehrter Lage mit niedergedrücktem Bolzen 50 von vorn über die Spitze 36 des Spießes 34 geschoben, bis der Bolzen 50 über den vorderen Einstich 42 hinweggeschoben ist und sich hinter diesem Einstich 42 befindet. Nun wird der Bolzen 50 freigegeben, so daß er unter dem Druck der Druckfeder 54 an dem Schaft des Spießes 34 anliegt. Der Griff 44 wird nach vorn weggezogen, bis der Bolzen 50 in den Bereich des vorderen Einstichs 42 gelangt und unter der Wirkung der Druckfeder 54 in diesen Einstich 42 einrastet. Damit ist der Griff 44 an dem vorderen Ende des Spießes 34 verriegelt und der Spieß 34 mit dem angeschraubten Drainageschlauch kann mittels des Griffes 44 vollständig durch die Schaumstoffplatte hindurch- und aus dieser herausgezogen werden.

Durch das Fenster 16 des Führungsrohres 10 kann beobachtet werden, wann das perforierte Ende des Drainageschlauches vollständig in die Schaumstoffplatte eingezogen ist. Ist diese Stellung erreicht, so wird der Spieß 34 von dem Drainageschlauch abgeschraubt. Die Arretierung des Scherengriffes mittels der Rastsperre 32 wird gelöst, der Scherengriff auseinandergespreizt und die Schaumstoffplatte mit dem eingezogenen Drainageschlauch kann aus der Halterung entnommen werden. Die Schaumstoffplatte wird nun entsprechend der zu versorgenden Wundfläche zugeschnitten, wobei auch der über die Schaumstoffplatte hinausragende perforierte Endabschnitt des Drainageschlauches abgeschnitten wird.

## Patentansprüche

1. Instrumentarium zum flächenparallelen Einziehen eines Drainageschlauches in eine Schaumstoffplatte mittels eines Spießes, auf dessen Ende der Drainageschlauch schraubbar ist, gekennzeichnet durch ein Führungsrohr (10) zum Hindurchführen des Spießes (34) mit dem Drainageschlauch und durch eine an dem Führungsrohr (10) angeordnete Halterung (18, 20), die die Schaumstoffplatte mit ihrer Schmalseite vor dem Führungsrohr (10) hält, wobei die Einziehrichtung mit der Achse der Innenbohrung (12) des Führungsrohres (10) fluchtet.

2. Instrumentarium nach Anspruch 1, dadurch gekennzeichnet, daß die Halterung (18, 20) die Schaumstoffplatte zumindest über den größten Teil der Länge des Einziehweges an ihrer Oberseite und Unterseite hält.

3. Instrumentarium nach Anspruch 2, dadurch gekennzeichnet, daß die Halterung aus zwei längs einer axialen Ebene voneinander getrennten Rohrschalen (18, 20) besteht, daß die erste Rohrschale (18) an dem Führungsrohr (10) befestigt ist und daß die zweite Rohrschale (20) gegen die erste Rohrschale (18) bewegbar und in bezug zu dieser arretierbar ist.

4. Instrumentarium nach Anspruch 3, dadurch gekennzeichnet, daß die zwei Rohrschalen (18, 20) sich zu einem mit dem Führungsrohr (10) axial fluchtenden Halterohr zusammenfügen, dessen Innendurchmesser im wesentlichen der Stärke der Schaumstoffplatte entspricht.

5. Instrumentarium nach Anspruch 4, dadurch gekennzeichnet, daß der Außendurchmesser des Führungsrohres (10) und der Außendurchmesser der Rohrschalen (18, 20) im wesentlichen gleich sind und daß die erste Rohrschale (18) als einstückige Verlängerung des Führungsrohres (10) ausgebildet ist.

6. Instrumentarium nach einem der Ansprüche 3 bis 5, dadurch gekennzeichnet, daß das Führungsrohr (10) mit der ersten Rohrschale (18) an einer Branche (28) eines Scherengriffes und die zweite Rohrschale (20) an der anderen Branche (30) des Scherengriffes angebracht sind und daß die Schwenkebene des Scherengriffes senkrecht zur Achse des Führungsrohres (10) ist.

7. Instrumentarium nach Anspruch 6, dadurch gekennzeichnet, daß der Scherengriff eine Rastsperre (32) zur Arretierung der Rohrschalen (18, 20) aufweist.

8. Instrumentarium nach einem der vorhergehenden Ansprüche, gekennzeichnet durch einen Griff (44) mit einer axial durchgehenden Bohrung (46) zum Hindurchführen des Spießes (34) mit dem Drainageschlauch, durch eine radial verschiebbar in den Querschnitt der Bohrung (46) eingreifende Verriegelung und durch einen Einstich (40) vor dem Ende des Spießes (34), in welchen die Verriegelung lösbar eingreift, um den Spieß (34) axial in dem Griff (44) zu verriegeln.

9. Instrumentarium nach Anspruch 8, dadurch gekennzeichnet, daß der Spieß (34) hinter seiner Spitze (36) einen Einstich (42) für den Eingriff der Verriegelung aufweist.

10. Instrumentarium nach Anspruch 8, dadurch gekennzeichnet, daß die Verriegelung einen in dem Griff (44) diametral verschiebbar gelagerten Bolzen (50) aufweist, der von einer Querbohrung (60) durchsetzt ist, daß der Bolzen (50) durch eine Feder (54) in eine Verriegelungsstellung vorgespannt ist, in welcher die Querbohrung (60) außer Fluchtung zu der Bohrung (46) des Griffes (44) ist, und daß der Bolzen (50) durch Druck auf sein aus dem Griff (44) ragendes Ende gegen die Kraft der Feder (54) in eine Freigabestellung drückbar ist, in welcher seine Querbohrung (60) mit der Bohrung (46) des Griffes (44) fluchtet.
